(19) Europäisches Patentamt
European Patent Office
Office européen des brevets

(11) Veröffentlichungsnummer: **0 226 109**
**A1**

(12) **EUROPÄISCHE PATENTANMELDUNG**

(21) Anmeldenummer: **86116658.5**

(22) Anmeldetag: **01.12.86**

(51) Int.Cl.⁴: **C 08 G 18/79**
C 08 G 18/02, C 08 G 18/16
C 07 D 251/34

(30) Priorität: **12.12.85 DE 3543925**

(43) Veröffentlichungstag der Anmeldung:
**24.06.87 Patentblatt 87/26**

(84) Benannte Vertragsstaaten:
**BE DE FR GB IT**

(71) Anmelder: **BAYER AG**
**Konzernverwaltung RP Patentabteilung**
**D-5090 Leverkusen 1 Bayerwerk(DE)**

(72) Erfinder: **Kerimis, Dimitrios, Dr.**
**Pulheimer Strasse 15**
**D-5000 Koeln 71(DE)**

(72) Erfinder: **Müller, Hanns Peter, Dr.**
**Im Kerberich 6**
**D-5068 Odenthal(DE)**

(54) **Verfahren zur Herstellung von Isocyanuratgruppen aufweisenden Polyisocyanaten und ihre Verwendung als Isocyanatkomponente zur Herstellung von Polyurethanen.**

(57) Ein neues Verfahren zur Herstellung von Isocyanuratgruppen aufweisenden Polyisocyanaten durch katalytische Trimerisierung eines Teils der Isocyanatgruppen von organischen Polyisocyanaten und Abbruch der Trimerisierungsreaktion, wobei man als Trimerisierungskatalysatoren quartäre Phosphoniumsalze aus tertiären Phosphinen und alkylierend wirkenden Estern von Säuren des Phosphors verwendet, sowie die Verwendung der Verfahrensprodukte, gegebenenfalls in von überschüssigen Polyisocyanaten befreiter Form und/oder gegebenenfalls in mit Blockierungsmitteln für Isocyanatgruppen blockierter Form als Isocyanatkomponente bei der Herstellung von Polyurethanen nach dem Isocyanat-Poly additionsverfahren.

EP 0 226 109 A1

BAYER AKTIENGESELLSCHAFT 5090 Leverkusen, Bayerwerk
Konzernverwaltung RP
Patentabteilung Wr/by-c

# Verfahren zur Herstellung von Isocyanuratgruppen aufweisenden Polyisocyanaten und ihre Verwendung als Isocyanatkomponente zur Herstellung von Polyurethanen

Die vorliegende Erfindung betrifft ein neues Verfahren zur Herstellung von Isocyanuratgruppen aufweisenden Polyisocyanaten durch katalytische Trimerisierung von monomeren Polyisocyanaten unter Verwendung von neuartigen Trimerisierungskatalysatoren, sowie die Verwendung der erfindungsgemäßen Verfahrensprodukte als Isocyanatkomponente bei der Herstellung von Polyurethanen nach dem Isocyanat-Polyadditionsverfahren.

Katalysatoren zur Trimerisierung organischer Isocyanate, insbesondere Polyisocyanate sind in großer Zahl bekannt (J.H. Saunders und K.C. Frisch, Polyurethanes Chemistry and Technology, S. 94 ff (1962)). Als Katalysatoren für die Trimerisierung sind starke organische Basen geeignet, so z.B. die alkalisch wirkenden Metallsalze von Carbonsäuren, Metallalkoholate, Metallphenolate, Alkalimetallcarbonate und tert. Amine.

Die Katalysatoren werden dabei häufig in Kombination eingesetzt oder zusammen mit weiteren Co-Katalysatoren wie Mono-N-substituierten Carbonaminsäureestern (A. Farkas und G.A. Mills, Advances in Catalysis, Vol. 13, 393 (1962)).

Bei den neueren Verfahren des Standes der Technik verwendet man spezielle organische Basen als Trimerisierungskatalysatoren, die in teilweise aufwendigen Syntheseverfahren hergestellt werden müssen.

So nimmt man z.B. zur Trimerisierung von aromatischen Polyisocyanaten Mannich-Basen (DE-OS 2 551 634 und DE-OS 2 641 380) oder tert. Phosphine, wobei im Falle der Phosphine zuerst Uretdione gebildet werden, die sich erst in zweiter Reaktionsphase zum Isocyanurat umsetzen (DE-OS 1 201 992). Zur Trimerisierung (cyclo)-aliphatischer Diisocyanate verwendet man neuerdings häufig organische Basen mit Betain-Struktur wie quartäre Amminiumhydroxide (EP-A 010 589 und EP-A 009 694), Aminimide (J.E. Kresta, R.J. Chang, S. Kathiriya und K.C. Frisch, Makromol. Chem. 180, 1081 (1979)) sowie Aziridinderivate in Kombination mit tert. Aminen (DE-OS 2 325 826).

Die DE-OS 3 227 489 beschreibt die Verwendung von quatären Ammoniumsalzen aus tertiären Aminen und alkylierend wirkenden Estern von Säuren des Phosphors als Trimerisierungskatalysatoren.

Mit der vorliegenden Erfindung wird eine neue Klasse von wertvollen Trimerisierungskatalysatoren zur Verfügung gestellt, die sich durch das gleichzeitige Vorliegen einer Reihe bemerkenswerter Vorteile auszeichnen:

1. Die neuen Katalysatoren eignen sich sowohl zur Trimerisierung von aromatischen als auch von aliphatischen Polyisocyanaten.

2. Die neuen Katalysatoren und auch ihre Umsetzungsprodukte mit den nachstehend näher beschriebenen Katalysatorgiften sind in den Ausgangs- und Endprodukten des nachstehend näher beschriebenen erfindungsgemäßen Verfahrens löslich, so daß sich aufwendige Trennungsoperationen erübrigen.

3. Die Trimerisierungsreaktion unter Verwendung der neuen Katalysatoren kann sowohl Lösungsmittel-frei als auch in Gegenwart von Lösungsmitteln bei vergleichsweise niedrigen Temperaturen durchgeführt werden, so daß als Verfahrensprodukte, leicht gelbe, klare Isocyanatgruppen aufweisende Polyisocyanate mit niedriger Viskosität entstehen.

4. Die schwach exotherme Trimerisierungsreaktion ist sowohl bei kontinuierlicher als auch diskontinuierlicher Fahrweise sicherheits- und verfahrenstechnisch leicht zu beherrschen.

5. Die Katalysatoren sind leicht und billig herstellbar und nahezu geruchlos.

Gegenstand der Erfindung ist ein Verfahren zur Herstellung von Isocyanuratgruppen aufweisenden Polyisocyanaten durch Trimerisierung eines Teils der Isocyanatgruppen von organischen Polyisocyanaten und Abbruch der Trimerisierungsreaktion durch Zugabe eines Katalysatorengifts, dadurch gekennzeichnet, daß man als Trimerisierungskatalysatoren quartäre Phosphoniumsalze aus tertiären Phosphinen und alkylierend wirkenden Estern von Säuren des Phosphors verwendet.

Gegenstand der Erfindung ist auch die Verwendung der nach diesem Verfahren erhältlichen, Isocyanuratgruppen aufweisenden Polyisocyanate, gegebenenfalls in von überschüssigen Ausgangspolyisocyanaten befreiter Form und/oder gegebenenfalls in mit Blockierungsmittel für Isocyanatgruppen blockierter Form als Isocyanatkomponente zur Herstellung von Polyurethanen nach dem Isocyanat-Polyadditionsverfahren.

Bei den neuen erfindungswesentlichen Trimerisierungskatalysatoren handelt es sich um quartäre, d.h. keine NH-Bindungen aufweisende Phosphoniumsalze, die Umsetzungsprodukte von (i) tertiären Phosphinen mit (ii) alkylierend wirkenden Estern von Säuren des Phosphors darstellen.

Bei der Katalysatorkomponente (i) handelt es sich um beliebige tertiäre Phosphine. Vorzugsweise weisen diese tertiären Phosphine ein Molekulargewicht von 76 bis 790, insbesondere von 202 bis 280 auf.

Typische derartige Phosphine sind Trimethylphosphin, Triethylphosphin, Tricyclohexylphosphin, Trioctylphosphin, Tristearylphosphin oder vorzugsweise Tri-n-butylphosphin, Triphenylphosphin und Triethylphosphin, wobei Tri-n-butylphosphin besonders geeignet ist.

Bei der Katalysatorenkomponente (ii) handelt es sich um neutrale, alkylierend wirkende Ester von anorganischen oder organischen Säuren des Phosphors. Hierunter sind vor allem die dieser Definition entsprechenden, gegebenenfalls inerte Substituenten aufweisenden Alkylester der Phosphorsäure und insbesondere von aromatischen oder aliphatischen Phosphonsäuren zu verstehen. Die der genannten Definition entsprechenden Alkylester anderer Säuren des Phosphors wie beispielsweise von phosphoriger Säure, Phosphinsäuren, phosphonigen und phosphinigen Säuren sind prinzipiell auch geeignet, jedoch weniger bevorzugt. Vorzugsweise werden die entsprechenden Alkylester mit 1 bis 4 Kohlenstoffatomen in den einzelnen Alkylresten eingesetzt.

Besonders bevorzugt sind Phosphonsäureester der Formel

$$R\text{-}\overset{\overset{\displaystyle O}{\|}}{P}(OR')_2$$

in welcher

R für einen, gegebenenfalls inerte Substituenten aufweisenden aromatischen Kohlenwasserstoffrest mit 6 bis 10 Kohlenstoffatomen, insbesondere Phenylrest oder einen aliphatischen Kohlenwasserstoffrest, insbesondere einen Alkylrest mit 1 bis 4 Kohlenstoffatomen steht, und

R' für gleiche oder verschiedene aliphatische Kohlenwasserstoffreste, insbesondere Alkylreste mit 1 bis 4 Kohlenstoffatomen stehen.

Typische Beispiele geeigneter Katalysatorkomponenten (ii) sind Triethylphosphat, Dimethyl-benzylphosphat, Trimethylphosphat, Benzolphosphonsäure-dimethylester, p-Toluolphosphonsäurediethylester, Methanphosphonsäuredimethylester, n-Butanphosphonsäurediethylester, Ethanphosphonsäurediethylester oder Ethanphosphonsäuredimethylester.

Bei der Herstellung der erfindungswesentlichen Katalysatoren werden die Einzelkomponenten (i) und (ii) in solchen Mengen miteinander umgesetzt, daß auf jedes Grammäquivalent an tertiärem Phosphor der Komponente (i) mindestens ein Mol der Komponente (ii) entfällt. Im allgemeinen wird ein beliebig hoher Überschuß der Komponente (ii) eingesetzt, der vorzugsweise nach Beendigung der Alkylierungsreaktion, beispielsweise durch Destillation, entfernt wird. Die Umsetzung der Einzelkomponenten (i) und (ii) erfolgt vorzugsweise in Substanz innerhalb des Temperaturbereichs von 50-200°C, vorzugsweise 80-180°C, während eines Zeitraums von ca. 0,5 bis 10 Stunden.

Oft ist es vorteilhaft, die Umsetzung unter Inertgasatmosphäre und/oder unter Druck durchzuführen. Die Reaktionsdauer und -temperatur richtet sich selbstverständlich in erster Linie nach der Reaktivität der jeweils zum Einsatz gelangenden Einzelkomponenten (i) und (ii).

Die so erhaltenen erfindungswesentlichen Trimerisierungskatalysatoren können selbstverständlich für die Herstellung beliebiger Isocyanurate eingesetzt werden. Dies bedeutet, daß sich die Katalysatoren nicht nur für das erfindungsgemäße Verfahren sondern auch beispielsweise

zur Herstellung von Isocyanuraten durch Trimerisierung von Monoisocyanaten eignen.

Für die Trimerisierungsreaktion werden die erfindungswesentlichen Katalysatoren in Substanz oder als 0,005- bis 95-, vorzugsweise 0,01- bis 70-gew.-%ige Lösungen eingesetzt.

Beispiele geeigneter Lösungsmittel sind Methanol, Ethanol, Propanol, Ethylenglykol, Propandiol-(1,2), Propandiol-(1,3), Butylenglykol, Glycerin oder Oligoethylen- bzw. -propylenglykole (Oligomerisierungsgrad 2 bis 6) (die Alkohole sollen einerseits die Ammoniumverbindungen gut lösen, andererseits aber mit dem Isocyanat noch einigermaßen mischbar sein und eine niedrige Viskosität besitzen).

Außerdem können auch aprotische, gegenüber Isocyanaten unreaktive Lösungsmittel eingesetzt werden, deren $E_T$-Wert (Chr. Reichardt, Lösungsmittel-Effekte in der organischen Chemie, Chem. Taschenbücher, Bd. 4, Verlag Chemie (Weinheim 1969) vorzugsweise im Bereich 33,5 bis 47 liegt. Außer Nitrilen, wie Acetonitril, Propionitril oder Benzonitril, Nitroverbindungen, wie Nitromethan oder Nitrobenzol, Kohlensäureestern, wie z.B. Ethylen- oder Propylencarbonat, oder Ketonen, wie Aceton, Acetophenon, Butyl-methyl-keton oder Isobutylmethyl-keton können selbst so unpolare Lösungsmittel wie Chlorkohlenwasserstoffe, z.B. Methylenchlorid, Chloroform, 1,1,1-Trichlorethan oder Trichlorethylen oder aromatische Kohlenwasserstoffe, wie Benzol, Toluol oder

Xylol oder Ester, wie Essigsäureethylester, Essigsäurebutylester oder Ethylenglykolmonomethyletheracetat eingesetzt werden.

Stark polare Lösungsmittel wie Dimethylformamid, N-Methylpyrrolidon, Tetramethylharnstoff oder Dimethylsulfoxid können zwar prinzipiell auch verwendet werden, ihr Einsatz ist jedoch nicht zu empfehlen, da sie einerseits nur schwierig von Nebenprodukten wie z.B. Aminen zu reinigen sind und andererseits im allgemeinen unerwünschte Nebenreaktionen von NCO-Gruppen katalysieren, so daß die erfindungsgemäßen Verfahrensprodukte bei Anwesenheit derartiger Lösungsmittel nicht lagerstabil sein würden.

Bei Verwendung von Hydroxylgruppen aufweisenden Lösungsmitteln entstehen beim erfindungsgemäßen Verfahren durch Reaktion mit einem Teil der Isocyanatgruppen des Ausgangspolyisocyanats Urethangruppen, was oftmals erwünscht ist, da derartige Urethangruppen einen cokatalytischen Effekt aufweisen. Die Menge an derartigen Lösungsmitteln, sofern es sich um einwertige Alkohole handelt, sollte jedoch so begrenzt werden, daß im Reaktionsgemisch maximal 2 Mol-%, bezogen auf die Isocyanatgruppen des Ausgangspolyisocyanats, an Hydroxylgruppen vorliegen. Oft ist es auch zweckmäßig, als Hydroxylgruppen aufweisende Lösungsmittel für die erfindungswesentlichen Katalysatoren mehrwertige Alkohole wie z.B. Ethylenglykol oder Glycerin einzusetzen, um die NCO-Funktionalität der erfindungsgemäßen Verfahrensprodukte nicht durch Urethanbildung zu reduzieren. Die Menge derartiger mehrwertiger Alkohole muß selbstverständlich jedoch so begrenzt werden, daß keine

in den Verfahrensprodukten schwerlöslichen Polyurethane entstehen. Für die Trimerisierungsreaktion werden die erfindungswesentlichen Katalysatoren im allgemeinen in Mengen von 0,005 bis 5, vorzugsweise 0,01 bis 2 Mol-%, bezogen auf zu trimerisierendes Ausgangspolyisocyanat einerseits und aus den Einzelkomponenten (i) und (ii) gebildeten Phosphoniumsalzen ohne Einbeziehung von gegebenenfalls noch vorliegendem überschüssigem, alkylierend wirkendem Ester andererseits, eingesetzt.

Falls aromatische Polyisocyanate ohne Mitverwendung von Lösungsmitteln trimerisiert werden, liegt die Menge der erfindungswesentlichen Katalysatoren vorzugsweise innerhalb des Bereichs von 0,01 bis 0,02 Mol-%, bei Verdünnung des aromatischen Ausgangspolyisocyanats mit einem geeigneten, aprotischen Lösungsmitel liegt die Menge des Katalysators im allgemeinen zwischen 0,01 und 0,1 Mol-%.

Bei Verwendung von Ausgangspolyisocyanaten mit ausschließlich aliphatisch gebundenen Isocyanatgruppen liegt die Menge des Katalysators im allgemeinen im Bereich von 0,05 bis 0,3 Mol-%, während bei Verwendung von Ausgangspolyisocyanaten mit cycloaliphatisch gebundenen Isocyanatgruppen vorzugsweise 0,3 bis 2,0 Mol-% des Katalysators eingesetzt werden, wobei sich alle Prozentangaben, wie oben dargelegt, auf das Ausgangspolyisocyanat einerseits und das Phosphoniumsalz andererseits beziehen.

Für die Trimerisierungsreaktion können beliebige organische Polyisocyanate als Ausgangsmaterialienm eingesetzt werden. Die neuen erfindungswesentlichen Katalysatoren

eignen sich insbesondere zur teilweisen Trimerisierung der Isocyanatgruppen von Diisocyanaten mit aromatisch, aliphatisch oder cycloaliphatisch gebundenen Isocyanatgruppen des Molekulargewichtsbereichs 140 bis 300, wie z.B. Tetramethylendiisocyanat, Hexamethylendiisocyanat, 1-Isocyanato-3,3,5-trimethyl-5-isocyanatomethyl-cyclohexan (Isophorondiisocyanat, abgekürzt: IPDI), 2,4- und/oder 2,6-Diisocyanatotoluol, 2,4'- und/oder 4,4'-Diisocyanatodiphenylmethan, 2,4'-Diisocyanatodicyclohexylmethan, 1-Methyl-2,4-diisocyanato-cyclohexan, Lysinesterdiisocyanaten, p-Xylylendiisocyanat oder beliebige Gemische derartiger Diisocyanate. Besonders gut geeignet sind auch Gemische der beispielhaft genannten aromatischen Diisocyanate mit beispielhaft genannten aliphatischen Diisocyanaten im Gewichtsverhältnis 1:3 bis 3:1. Auch höherfunktionelle Polyisocyanate wie beispielsweise Polyisocyanatgemische, die durch Phosgenierung von Anilin/Formaldehyd-Kondensaten entstehen, können beim erfindungsgemäßen Verfahren als Ausgangspolyisocyanat eingesetzt werden. Grundsätzlich, jedoch weniger bevorzugt, ist es auch möglich, NCO-Präpolymere, d.h. Umsetzungsprodukte von überschüssigen Mengen der beispielhaft genannten Diisocyanate mit mindestens difunktionellen Verbindungen mit gegenüber Isocyanatgruppen reaktionsfähigen Gruppen als Ausgangspolyisocyanate beim erfindungsgemäßen Verfahren einzusetzen. Grundsätzlich können beim erfindungsgemäßen Verfahren auch Gemische aus Diisocyanaten und Monoisocyanaten als Ausgangsmaterialien eingesetzt werden, um so zu interessanten Isocyanuratgruppen aufweisenden Polyisocyanaten mit einer gezielt erniedrigten NCO-Funktionalität zu gelangen. Hierbei werden die Di- und Monoisocyanate im allgemeinen in einem Molverhältnis Diisocyanat:Monoisocyanat von 1,5:1 bis 2,5:1 eingesetzt. Geeignete Monoisocyanate sind beispielsweise aliphatische Monoisocyanate mit 1 bis 18, vorzugsweise 4 bis 8 Kohlenstoffatomen wie Methylisocyanat, n-Butylisocyanat, n-Octylisocyanat oder Stearylisocyanat oder aromatische Monoisocyanate wie insbesondere Phenylisocyanat. Bevorzugte Ausgangspolyisocyanate für das erfindungsgemäße Verfahren sind 2,4- und/oder 2,6-Diisocyanatotoluol, Hexamethylendiisocyanat und IPDI.

Die Trimerisierungsreaktion kann in Abwesenheit oder in Gegenwart von gegenüber Isocyanatgruppen inerten Lösungsmitteln durchgeführt werden. Als Lösungsmittel für das erfindungsgemäße Verfahren eignen sich beliebige, gegenüber Isocyanatgruppen inerte Lösungsmittel bzw. Lösungsmittelgemische, deren Siedepunkt im weiten Bereich von ca. 50° C/1013 mbar bis 250° C/13,3 mbar liegt. Je nach Anwendungsbereich der erfindungsgemäßen Verfahrensprodukte können niedrig- bis mittelsiedende Lösungsmittel oder hochsiedende angewandt werden. Bevorzugt einzusetzende Lösungsmittel sind beispielsweise Ester wie Ethylacetat, Butylacetat, Ethylenglykol-monomethylether-acetat, Ethylenglykol-monoethylether-acetat odr Ketone wie z.B. Aceton, Methylethylketon, Methylisobutylketon, Cyclohexanon oder Methoxyhexanon. Auch Phthalsäureester wie z.B. Dibutylphthalat, Butylbenzylphthalat oder Phosphorsäureester wie Trikresylphosphat oder auch Alkylsulfonsäureester des Phenols und des Kresols sind geeignet. In Verdünnungsmitteln wie z.B. Toluol, Xylol oder höheren

Aromaten besteht oft nur eine begrenzte Löslichkeit, so daß höhere Zusätze solcher Verdünnungsmittel zu Trübungen und Ausfällungen in den Reaktionsprodukten führen können.

Das bei der Durchführung der Trimerisierungsreaktion eingesetzte Lösungsmittel bzw. die eingesetzte Lösungsmittelmenge braucht nicht mit dem Lösungsmittel bzw. der Lösungsmittelmenge identisch zu sein, die in den erfindungsgemäßen Verfahrensprodukten bei ihrer erfindungsgemäßen Verwendung vorliegen. So kann man nach Beendigung des erfindungsgemäßen Verfahrens das eingesetzte Lösungsmittel bzw. Lösungsmittelgemisch selbstverständlich ganz oder teilweise destillativ entfernen und ganz oder teilweise urch ein anderes Lösungsmittel ersetzen. Lösungsmittlfrei hergestellte Produkte können selbstverständlich nachträglich in den obengenannten Lösungsmitteln gelöst werden.

Die Trimerisierungsreaktion wird im allgemeinen im Temperaturbereich von 0 bis 200° C, vorzugsweise zwischen 10 und 100° C und besonders bevorzugt zwischen 25 und 80° C durchgeführt. Bei der Durchführung des erfindungsgemäßen Verfahrens in Gegenwart eines Lösungsmittels setzt man im allgemeinen das zu trimerisierende Ausgangspolyisocyanat und das betreffende Lösungsmittel in einem Gewichtsverhältnis von 1:4 bis 4:1, vorzugsweise 1:2 bis 2:1, besonders bevorzugt 0,8:1,2 bis 1,2:0,8 entsprechenden Mengen ein.

Die zu wählende Katalysatormenge richtet sich, wie oben ausgeführt, nach der Art des Ausgangspolyisocyanats und selbstverständlich auch nach der Reaktionstemperatur, bei welcher die Trimerisierung durchgeführt werden soll. Sie kann in einem einfachen orientierenden Vorversuch zuverlässig ermittelt werden. Im allgemeinen ist die Katalysatorenkonzentration gegenüber dem lösungsmittelfreien Verfahren bei Verwendung von Lösungsmitteln um den Faktor 5 bis 15 zu erhöhen.

Die Trimerisierungsreaktion kann beispielsweise nach folgenden Varianten durchgeführt werden:

1. Der Katalysator bzw. seine Lösung in einem der hierfür geeigneten Lösungsmittel wird dem zu trimerisierenden Polyisocyanat ohne Mitverwendung eines Hilfslösungsmittels für die Trimerisierungsreaktion bei Raumtemperatur zudosiert, worauf im allgemeinen die Trimerisierungsreaktion spontan anspringt. Die Reaktionstemperatur wird anschließend durch äußeres Heizen auf der gewünschten Reaktionstemperatur gehalten, bis die Trimerisierungsreaktion durch Zugabe eines Katalysatorengiftes abgebrochen wird.

2. Das zu trimerisierende Polyisocyanat wird in einem Lösungsmittel der oben beispielhaft genannten Art vorgelegt; anschließend wird diese Lösung mit dem Trimerisierungskatalysator bzw. seiner Lösung versetzt. Auch bei dieser Variante des erfindungsgemäßen Verfahrens wird die Temperatur des Reaktionsgemisches gegebenenfalls durch äußeres Heizen innerhalb der genannten Bereiche eingestellt.

Auch hier erfolgt der Abbruch der Trimerisierungsreaktion beim Erreichen des erwünschten Trimerisierungsgrades durch Zugabe eines Katalysatorengiftes.

Bei allen beispielhaft genannten Varianten wird die Trimerisierungsreaktion im allgemeinen bei Erreichen eines Trimerisierungsgrades (Trimerisierungsgrad = Prozentsatz der trimerisierten Isocyanatgruppen, bezogen auf Gesamtmenge der im Ausgangspolyisocyanat vorliegenden Isocyanatgruppen) von 10 bis 70 % abgebrochen. Bei der lösungsmittelfreien Durchführung des erfindungsgemäßen Verfahrens unter anschließender Entfernung von überschüssigem Ausgangspolyisocyanat, beispielsweise im Dünnschichtverdampfer, liegt der Trimerisierungsgrad im allgemeinen zwischen 10 und 40 %. Bei der Durchführung des erfindungsgemäßen Verfahrens in Gegenwart von Lösungsmitteln ohne anschließende Entfernung von nicht-umgesetztem Ausgangspolyisocyanat liegt der Trimerisierungsgrad im allgemeinen zwischen 50 und 70 %.

Geeignete Katalysatorengifte sind beispielsweise beliebige Säurehalogenide, insbesondere Säurechloride, wie z.B. Acetylchlorid, Benzoylchlorid, Terephthaloyldiol, Phthaloyldichlorid, Trichloracetylchlorid, Phosphortrichlorid oder Phosphortribromid oder auch, jedoch weniger bevorzugt, starke, den Katalysator neutralisierende und damit desaktivirende Säuren wie z.B. Schwefelsäure, Phosphorsäure, Chlorwasserstoff, Toluolsulfonsäure, Methansulfonsäure, Chlorsulfonsäure, Nona-fluorbutansulfonsäure oder Dibutylphosphorsäure. Weiterhin ist

Tosylisocyanat als Stopper geeignet. Zur Desaktivierung des Katalysators ist es ausreichend, wenn dem Reaktionsgemisch 100 bis 110 Äquivalentprozent, d.h. im Falle von monofunktionellen Katalysatorengiften 100 bis 110 Mol-%, bezogen auf die im Katalysator vorliegenden quartären Phosphoniumgruppen, des Katalysatorgiftes zugesetzt werden.

Die erfindungsgemäßen Verfahrensprodukte können insbesondere im Falle der lösungsmittelfreien Durchführung der Trimerisierungsreaktion in an sich bekannter Weise, beispielsweise durch Dünnschichtdestillation von überschüssigem, nicht-umgesetztem Ausgangspolyisocyanat befreit werden, so daß Isocyanuratgruppen aufweisende Polyisocyanate mit einem Gehalt an monomeren Ausgangsdiisocyanaten unter 3 Gew.-%, vorzugsweise unter 0,7 Gew.-%, erhältlich sind.

Die erfindungsgemäßen Verfahrensprodukte können selbstverständlich in an sich bekannter Weise mit geeigneten Blockierungsmitteln für Isocyanatgruppen wie z.B. Phenol, ε-Caprolactam, Malonsäurediethylester oder Acetessigsäureethylester blockiert werden.

Die erfindungsgemäßen Verfahrensprodukte bzw. ihre durch die genannte Blockierungsreaktion erhaltenen Derivate stellen wertvolle Ausgangsmaterialien zur Herstellung von Polyurethankunststoffen nach dem Isocyanat-Polyadditionsverfahren dar. Sie eignen sich insbesondere als Isocyanatkomponente in Zweikomponentenpolyurethanlacken.

Die Möglichkeit, die erfindungsgemäße Reaktion durch Zugabe von Säurechloriden abzustoppen, konnte nicht vorhergesehen werden, da eigentlich erwartet werden mußte, daß quartäre Phosphoniumsalze der Art der erfindungswesentlichen Katalysatoren gegenüber Säurechloriden weitgehend inert sind.

In den nachfolgenden Beispielen beziehen sich alle Prozentangaben, soweit nicht anders lautend ausgeführt, auf Gewichtsprozente.

Beispiele

In den nachfolgenden Beispielen werden die folgenden erfindungsgemäßen Trimerisierungskatalysatoren eingesetzt:

Katalysator I:

40,4 Gew.-Teile Tri-n-butylphosphin und
121 Gew.-Teile Methanphosphonsäuredimethylester werden vermischt und unter leichter Stickstoff-Durchleitung 16 Stunden bei 100° C gerührt. Anschließend wird der überschüssige Methanphosphonsäuredimethylester im Vakuum abdestilliert.

Man erhält 54 Gew.-Teile einer mittelviskosen farblosen und geruchlosen Flüssigkeit.

Katalysator II:

40,4 Gew.-Teile Tri-n-butylphosphin und
56 Gew.-Teile Trimethylphosphat werden vermischt und unter leichter Stickstoff-Durchleitung 16 Stunden bei 100° C gerührt. Anschließend wird das überschüssige Trimethylphosphat im Vakuum abdestilliert.

Man erhält 65 Gew.-Teile einer mittelviskosen farblosen und geruchlosen Flüssigkeit.

Beispiel 1

500 Gew.-Teile 2,4-Toluylendiisocyanat werden in 500 Gew.-Teile wasserfreiem Butylacetat gelöst und bei Zimmertemperatur mit 2,5 Gew.-Teile einer 10 gew.-%igen Lösung des Katalysators I in Ethanol versetzt. Das Reaktionsgemisch wird anschließend 60 Stunden bei Zimmertemperatur gerührt.

Schließlich wird die Trimerisierungsreaktion mit 4 Gew.-Teilen einer 3 gew.-%igen Lösung von Benzoylchlorid in wasserfreiem Butylacetat gestoppt und das Reaktionsgemisch 1 Stunde bei 60°C nachgerührt.

Man erhält eine klare, leicht gelbe Lösung. Die Spezifikationen der Lösung sind:

| | |
|---|---|
| NCO-Gehalt: | 7,7 Gew.-% |
| freies 2,4-Toluylendiisocyanat: | 0,35 Gew.-% |
| Viskosität $\eta_{23^\circ C}$: | 1700 mPas |

Beispiel 2

800 ml Hexamethylendiisocyanat (HDI) werden bei Raumtemperatur vorgelegt und unter leichter Stickstoffüberleitung mit 12 ml einer 0,5 m Lösung des Katalysators I in Ethylhexanol versetzt. Nach ca. 18-20 Stunden wird bei einem NCO-Gehalt von 40 bis 41 % mit einer äquivalenten Menge Tosylisocyanat gestoppt und das nahezu farblose Rohprodukt gedünnschichtet.

Man erhält ein klares und leicht gelbes Sumpfprodukt mit folgender Spezifikation:

NCO-Gehalt: 22,5 Gew.-%

Viskosität $\eta_{21^\circ C}$: 1100 mPas

Beispiel 3

250 Gew.-Teile Isophorondiisocyanat (IPDI) werden mit 5 Gew.-Teilen des Katalysators I versetzt und bei 100° C (Badtemperatur) gerührt. Nach 20 Stunden ist bei konstanter Innentemperatur der NCO-Gehalt auf 30 % gesunken.

Beispiel 4

100 Gew.-Teile Phenylisocyanat werden in 100 Gew.-Teilen Toluol gelöst und mit 1 Gew.-Teil des Katalysators II versetzt und bei 60° C gerührt. Nach kurzer Zeit steigt die Temperatur auf 70° C und es beginnt die Ausfällung des Trimerisats. Zur Vervollständigung der Reaktion rührt man noch 5 Stunden bei 60° C, saugt das Kristallisat ab und trocknet im Vakuum.

Man erhält 91 Gew.-Teile Triphenylisocyanurat mit einem Schmelzpunkt von 284° C.

Patentansprüche

1. Verfahren zur Herstellung von Isocyanuratgruppen aufweisenden Polyisocyanaten durch Trimerisierung eines Teils der Isocyanatgruppen von organischen Polyisocyanaten und Abbruch der Trimerisierungsreaktion durch Zugabe eines Katalysatorgifts, dadurch gekennzeichnet, daß man als Trimerisierungskatalysatoren quartäre Phosphoniumsalze aus tertiären Phosphinen und alkylierend wirkenden Estern von Säuren des Phosphors verwendet.

2. Verfahren gemäß Anspruch 1, dadurch gekennzeichnet, daß man als Trimerisierungskatalysatoren quartäre Phosphoniumsalze verwendet, welche Umsetzungsprodukte von

(i) tertiären Phosphinen mit

(ii) Phosphonsäureestern der Formel

$$R-\overset{\overset{\displaystyle O}{\|}}{P}(OR')_2$$

in welcher

R für einen, gegebenenfalls inerte Substituenten aufweisenden Phenylrest oder einen Alkylrest mit 1 bis 4 Kohlenstoffatomen steht und

R' einen Alkylrest mit 1 bis 4 Kohlenstoffatomen bedeutet,

darstellen.

3. Verfahren gemäß Anspruch 1 und 2, dadurch gekennzeichnet, daß man als Katalysatorengifte organische Säurechloride oder Tosylisocyanat verwendet.

4. Verfahren gemäß Anspruch 1 bis 3, dadurch gekennzeichnet, daß man die Umsetzung im Temperaturbereich von 10 bis 100°C unter Verwendung einer Katalysatormenge von 0,01 bis 2 Mol-%, bezogen auf die Menge des teilweise zu trimerisierenden Polyisocyanats, durchführt.

5. Verwendung der gemäß Anspruch 1 bis 4 erhältlichen, Isocyanuratgruppen aufweisenden Polyisocyanate, gegebenenfalls in von überschüssigen Ausgangspolyisocyanaten befreiter Form und/oder gegebenenfalls in mit Blockierungsmitteln für Isocyanatgruppen blockierter Form als Isocyanatkomponente zur Herstellung von Polyurethanen nach dem Isocyanat-Polyadditionsverfahren.

Europäisches Patentamt

**EUROPÄISCHER RECHERCHENBERICHT**

0226109

Nummer der Anmeldung

EP 86116658.5

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,Y | DE - A1 - 3 227 489 (BAYER AG)<br>* Ansprüche *<br>-- | 1-5 | C 08 G 18/79<br>C 08 G 18/02<br>C 08 G 18/16<br>C 07 D 251/34 |
| Y | DE - A - 1 954 093 (MOBAY CHEMICAL COMPANY)<br>* Anspruch 1; Seite 2, Zeilen 6-10; Seite 4, Zeilen 13-27; Beispiel 1 *<br>-- | 1-4 | |
| A | CHEMICAL ABSTRACTS, vol. 93, no. 13, 29. September 1980, Columbus, Ohio, USA<br>D.M.YUKHNOVICH et al. "Triarylisocyanurate"<br>Seite 669, Spalte 2, Zusammenfassung Nr. 132 518e<br>-- | 1,2 | |
| A | US - A - 4 066 629 (EDELMAN)<br>* Anspruch 1 *<br>---- | 1,2 | **RECHERCHIERTE SACHGEBIETE (Int. Cl.4)**<br>C 08 G 18/00<br>C 07 D 251/00 |

Der vorliegende Recherchenbericht wurde für alle Patentansprüche erstellt.

| Recherchenort | Abschlußdatum der Recherche | Prüfer |
|---|---|---|
| WIEN | 27-02-1987 | WEIGERSTORFER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82